# EUROPÄISCHE PATENTSCHRIFT

(11) **EP 1 408 922 B1**
(45) Veröffentlichungstag und Bekanntmachung des Hinweises auf die Patenterteilung: **02.12.2009**
(21) Anmeldenummer: 02754820.5
(22) Anmeldetag: 03.07.2002
(51) Int. Cl.: A61K 8/34, A61K 8/81, A61Q 19/00

(54) **VERFAHREN ZUR HERSTELLUNG VON DESINFIZIERENDEN HAUT- UND HANDPFLEGEGELEN**
METHOD FOR PRODUCING DISINFECTANT SKIN AND HAND CARE GELS
PROCEDE DE PRODUCTION DE GELS DESINFECTANTS POUR LE SOIN DE LA PEAU ET DES MAINS

(30) Priorität: 06.07.2001 DE 10132382
(43) Veröffentlichungstag der Anmeldung: 21.04.2004
(73) Patentinhaber: Evonik Stockhausen GmbH, 47805 Krefeld (DE)
(72) Erfinder: VEEGER, Marcel, 47574 Goch (DE); KLOTZ, Andreas, 41516 Grevenbroich (DE); NAUELS, Bernd, 47906 Kempen (DE); BLÄSER, Edeltraud, 47803 Krefeld (DE)
(86) Internationale Anmeldenummer: PCT/EP2002/007386
(87) Internationale Veröffentlichungsnummer: WO 2003/003998

(56) Entgegenhaltungen:
- EP-A- 0 604 848
- US-A- 4 956 170
- DATABASE WPI Section Ch, Week 199344 Derwent Publications Ltd., London, GB; Class A96, AN 1993-348345 XP002217807 & JP 05 255078 A (NIPPON SEIYAKU KK), 5. Oktober 1993 (1993-10-05)

## Beschreibung

Die Erfindung betrifft ein Verfahren zur Herstellung von desinfizierenden Hautpflegegelen, insbesondere desinfizierende Handpflegegele mit pflegenden Komponenten.

Desinfektionsmittel dienen der Bekämpfung pathogener Mikroorganismen wie z.B. Bakterien, Viren, Sporen, Pilze etc. und ihr Einsatz ist in vielen Bereichen unverzichtbar bzw. deren Verwendung wird in vielen Ländern vom Gesetzgeber ausdrücklich verlangt.

Üblicherweise werden Desinfektionsmittel entsprechend ihres Einsatzgebietes gegliedert und man unterscheidet je nach Verwendungszweck Antiseptika zur Wund-, Haut-, Stuhl- und Sputumdesinfektion sowie Instrumentendesinfektion, Wäsche- und Flächendesinfektionsmittel, insbesondere aber auch Haut- und Händedesinfektionsmittel.

Das Anwendungsgebiet der vorgenannten Desinfektionsmittel ist medizinisch indiziert und dient der Prävention von Infektionen in Krankenhäusern, Arzt- und Zahnarztpraxen, in öffentlichen Bereichen wie Schulen, Kindergärten und Pflegeeinrichtungen z.B. Altenheimen, Sanatorien etc., aber auch Sportstätten sowie in anderen Bereichen, in denen Infektionen übertragen werden können. Neben dem Einsatz von Desinfektionsmitteln in der Lebensmittelindustrie und Pharmaindustrie ist nicht nur allgemein deren Einsatz am Arbeitsplatz oder in Wohnungen, sondern auch deren Verwendung in Dienstleistungseinrichtungen wie z.B. Wäschereien und Küchen zu nennen, deren Produkte direkt an Patienten oder Verbraucher geliefert wird.

Der Nachweis der Wirksamkeit solcher desinfizierender Mittel für eines oder mehrere der oben genannten Anwendungsgebiete erfolgt nach eingehender Prüfung dieser Mittel unter Zugrundelegung normierter Prüfverfahren, die z.B. in Deutschland den Richtlinien der Deutschen Gesellschaft für Hygiene und Mikrobiologie (DGHM) bzw. den Richtlinien des französischen Normungsinstitut AFNOR (Association Française de Normalisation) entnommen sind. Beispielhaft sei auf die nachfolgenden Normen hingewiesen:

| | |
|---|---|
| DIN EN 1040 | Chemische Desinfektionsmittel und Antiseptika (Basistest) |
| DIN EN 1276 | Chemische Desinfektionsmittel und Antiseptika Bakterizide Wirkung in den Bereichen Lebensmittel, Industrie, Haushalt und öffentliche Einrichtungen |
| DIN EN 1499 | Desinfizierende Händewaschung |
| DIN EN 1500 | Hygienische Händedesinfektion |
| DIN EN 12054 | Chemische Desinfektionsmittel und Antiseptika Produkte für die hygienische und chirurgische Händedesinfektion und Händewaschung - Bakterizide Wirkung |
| DIN EN 12791 | Chirurgische Händedesinfektionsmittel |
| | |
| AFNOR T 72 300 | Bakterizide Wirksamkeit von Antiseptika und Desinfektionsmitteln, die als Flüssigkeit, gemischt in Wasser angewendet werden |
| AFNOR T 72 170 | Bakterizide Wirksamkeit in Gegenwart von Störstoffen |
| NF EN 1040 | Bakterizide Wirksamkeit von Antiseptika und chemischen Desinfektionsmitteln |
| NF EN 1275 | Fungizide Wirksamkeit von Antiseptika und chemischen Desinfektionsmitteln |

Antiseptika unterliegen als Arzneimittel darüber hinaus gesetzlich geregelter Zulassungs- und Registrierungsverfahren.

Wie z.B. der DE-A-43 28 828 entnommen werden kann, gibt es verschiedene Verfahren zur Durchführung der Händedesinfektion. Explizit werden dort die in Deutschland übliche alkoholische Händedesinfektion sowie die waschende Händedesinfektion (Scrub-Methode) genannt. Produkte, die u.a. für die Händedesinfektion bestimmt sind, müssen wenigstens den in den oben genannten Normen aufgeführten Mindestanforderungen genügen, wenn sie entsprechend der erfüllten Normen zertifiziert und als Präparate in der Desinfektionsliste der DGHM aufgenommen werden sollen.

FR-A-2785537 offenbart Pflege- und Feuchthaltewirkstoffe enthaltende desinfizierende Haut- und Handpflege-Zusammensetzungen, die in Gelform vorliegen und über 50 vol % Alkohol enthalten.

Im Markt erhältliche Händedesinfektionsmittel bestehen üblicherweise aus Alkohol oder Mischungen aus Alkoholen sowie optional Pflegewirkstoffen, die nach dem Verdunsten der Alkoholkomponente auf der Haut verbleiben, z.B. nichtflüchtige antimikrobiell wirksame Substanzen und/oder übliche Hautpflegestoffe sowie gegebenenfalls weiteren Hilfsstoffen. Dient lediglich die Alkoholkomponente als antimikrobieller Wirkstoff, so ist der Alkoholgehalt in dem Mittel so zu wählen, daß auch bei Verdunsten eines Teiles des Alkohols eine desinfizierende Wirkung gewährleistet ist. In diesem Zusammenhang ist bekannt, daß dies der Fall ist für ethanolische Zusammensetzungen mit einem Alkoholgehalt von mindestens 52 Gew. -%.

Im Hinblick auf die Nachteile alkoholischer Desinfektionslösungen bei der Applikation für die Händedesinfektion, insbesondere ist die Dosierung schwierig, weil die benötigte Desinfektionsmittelmenge vielfach nicht gleichmäßig über die Hände verteilt werden kann, da die Alkohollösung sehr leicht von den Händen tropft, sind solchen Desinfektionslösungen Verdickungsmittel zugesetzt worden, um die Viskosität der Mittel zu erhöhen. Als Stand der Technik ist hier beispielhaft die EP-B-0 604 848 zu nennen, die eine schnelltrocknende Desinfektionsmittelzusammensetzung zum Gegenstand hat. Als Verdickungsmittel wird eine Kombination aus Carboxyvinylpolymeren und Hydroxypropylmethylcellulose verwendet, wobei das Gesamtgewicht der beiden Komponenten in der Desinfektionsmittelzusammensetzung nicht größer als 3 Gew.-% ist.

Antimikrobielle alkoholische Gelzusammensetzungen zur Händedesinfektion, die Feuchthaltemittel und hautpflegende Substanzen enthalten, werden in der US 4 956 170 beschrieben. In diesen Zusammensetzungen werden vernetzte teilneutralisierte bzw. neutralisierte Acrylsäurepolymeren als Verdickungsmittel eingesetzt. Als antibakterielles Mittel dient in diesen Zusammensetzungen 60 bis 75 Gew.-% Alkohol wie z.B. Ethanol, Isopropropylalkohol oder Mischungen hiervon. Im Hinblick auf die in diesen Gelzusammensetzungen enthaltenen Weichmacher (Emollientien) wie Petrolatum und anderen Mineralölprodukten, die in kosmetischen Präparaten eingesetzt werden können sowie weiteren hydrophoben Inhaltsstoffen, die in Kosmetika aber auch in Desinfektionsmittel unbedenklich eingesetzt werden können, ist ausgeführt worden, daß die Verwendung solcher Inhaltsstoffe in alkoholischen Gelzusammensetzungen mit hohem Alkoholgehalt für die Stabilität dieser Gele sehr nachteilig ist, weil die Gele bei der Lagerung im Laufe der Zeit ihre Viskosität und damit ihre Stabilität verlieren und die Zusammensetzungen zerfließen. Darüber hinaus ist festgestellt worden, daß die Gelstabilität mit zunehmendem Alkoholgehalt leidet, insbesondere bei Alkoholgehalten über 60 Gew.-%.

Zur Verbesserung der Stabilität wurden daher den Gelzusammensetzungen mit Hydroxypropylguaran ein weiteres Verdickungsmittel zugesetzt, wobei bei einem Alkoholehalt von 60 Gew.-% nur hochsubstituiertes Hydroxypropylguaran eine adäquate Gelstabilität gewährleisten kann, während dies bei einem Zusatz von Hydroxypropylguaran mit einem geringeren Substitutionsgrad nicht gewährleistet ist.
Hohe Alkoholgehalte in Gelzusammensetzungen mit Alkohol als einziger Wirkkomponente sind jedoch unverzichtbar, damit diese Gele, insbesondere als Desinfektionsmittel für die Händedesinfektion zertifiziert werden können.

Aufgabe der vorliegenden Erfindung war es daher, ein einfaches, wirtschaftliches Herstellungsverfahren bereitzustellen, das die Herstellung von stabilen desinfizierenden Haut- und Handpflegegelen mit hohem Alkoholgehalt, insbesondere desinfizierenden Handpflegegelen mit pflegenden Komponenten ermöglicht.

Die Aufgabe wird gelöst durch ein Verfahren zur Herstellung von desinfizierenden Haut- und Handpflegegelen, die
a.) mindestens 60 Gew-%, bezogen auf die Gesamtmenge des Geles, einer alkoholischen Wirkstoffkomponente bestehend aus einer Alkoholmischung, die einen Anteil von mindestens 5 Gew.-%, bezogen auf die Gesamtmenge des Geles, wenigstens eines Alkohols der allgemeinen Formel

   **R-OH**

   in der R für einen aliphatischen, linearen oder verzweigten Kohlenwasserstoffrest mit 3 bis 8 Kohlenstoffatomen steht, enthält,
b.) mindestens ein Verdickungsmittel auf Basis von Polyacryl- und Polymethacrylsäureverbindungen sowie gegebenenfalls weiteren Verdickungsmitteln
c.) 0 bis 5 Gew.-%, bezogen auf die Gesamtmenge des Geles, mindestens einen Pflege-und/oder Feuchthaltewixkstoff sowie
d.) Wasser enthalten, wobei
   1.) mindestens ein Acrylsäurepolymerisat zu einer Vorlage von Wasser gegeben und anschließend das Polymerisat dispergiert wird, bis eine milchigtrübe, homogene Dispersion entsteht;
   2.) eine alkoholische Pflegephase, enthaltend eine alkoholische Lösung sowie gegebenenfalls Pflege- und/oder Feuchthaltewirkstoffe, fraktioniert oder komplett zur Dispersion hinzugefügt wird,
   3.) die gebildete Polymerisatlösung mit mindestens einem Neutralisationsmittel neutralisiert wird, dadurch gekennzeichnet, daß mindestens 5 Gew.-%, bezogen auf die Gesamtmenge des Geles, wenigstens eines Alkohols der allgemeinen Formel

      **R-OH**

      in der R für einen aliphatischen, linearen oder verzweigten Kohlenwasserstoffrest mit 3 bis 8 Kohlenstoffatomen steht, nach oder während der Neutralisation zur Gesamtmischung gegeben werden, und die Menge des eingesetzten Verdickungsmittel so gewählt wird, daß das resultierende Haut- und Handpflegegel eine Viskosität von 4 bis 10 Pa•s bei 25° C (4000 bis 10 000 Centipoise) aufweist.

Die erfindungsgemäß einzusetzende alkoholische Wirkstoffkomponente, die zu mindestens 60 Gew.-%, bezogen auf die Gesamtmenge des Geles, in den nach dem erfindungsgemäßen Verfahren hergestellten desinfizierenden Haut- und Handpflegegelen enthalten sein muß und die mindestens 5 Gew.-%, bezogen auf die Gesamtmenge des Geles, wenigstens eines Alkohols der allgemeinen Formel

**R-OH**

in der R für einen aliphatischen, linearen oder verzweigten Koblenwasserstoffrest mit 3 bis 8 Kohlenstoffatomen steht, enthält, weist eine Hauptkomponente aus Methanol, Ethanol, n- Propanol, Isopropylalkohol oder eine Mischung hiervon auf.

Als Hauptkomponente wird vorzugsweise technischer Alkohol, d.h. Alkohol, der mindestens 92,4 Gew.% Ethanol enthält oder absoluter Alkohol (mindestens 99,7 Vol.-% Ethanol), verwendet. Besonders bevorzugt ist Neutralalkohol, mit Phthalsäurediethylester vergällt - Sorte 611 (Ethanol, 95,6 Vol.-%, vergällt mit ca. 0,6% Diethylphthalat).

Als Alkohole der allgemeinen Formel

**R-OH**

in der R für einen aliphatischen, linearen oder verzweigten Kohlenwasserstoffrest mit 3 bis 8 Kohlenstoffatomen steht, die allein oder in Mischungen eingesetzt werden, können beispielsweise 1-Propanol, 2-Propanol (Isopropylalkohol), 1-Butanol, Isobutylalkohol, tert. Butylalkohol, die Amylalkohole 1-, 2-, 3- Pentanol oder Neopentylalkohol verwendet werden, wobei Isopropylalkohol besonders bevorzugt ist.

Als erfindungsgemäß einzusetzende Verdickungsmittel sind kommerziell erhältliche vollsynthetische Verdickungsmittel auf Basis von Polyacryl- und Polymethacrylverbindungen zu nennen. Besonders bevorzugt sind Acrylsäurepolymerisate, die z.B. unter der Marke CARBOPOL ® von der Fa. BFGoodrich Chemical Europe, Brüssel (BE) wie z.B. CARBOPOL ETD 2020 erhältlich sind. Den vorgenannten Verdickungsmitteln können weitere übliche Verdickungsmittel auf Basis von Vinylpolymeren, Polycarbonsäuren, Polyiminen, Polyamiden und Polyether hinzugefügt sein. Darüber hinaus können auch Carboxymethylcellulosen und Celluloseether, Kernmehlether, insbesondere aber Hydroxyethyl- und Hydroxypropylcellulosen, die unter den Marken Natrosol® bzw. Klucel® von der Fa. Aqualon GmbH & Co.KG bezogen werden können sowie auch Xanthane der Marke Keltrol® T (Kelco Division, San Diego, Kalifornien) eingesetzt werden. Als natürliche Verdickungsmittel können u.a. Guar-Mehl und Johannesbrotbaumkernmehl verwendet werden.

Die Menge des eingesetzten Verdickungsmittel wird hierbei so gewählt, daß das resultierende Haut- und Handpflegegel eine Viskosität von 4000 bis 10 000 Centipoise (4 bis 10 Pa·s bei 25 °C) aufweist. Üblicherweise beträgt die Gesamtmenge des Verdickungsmittels 0,1 bis 3 Gew.-% (bezogen auf die Gesamtmenge des Geles).

Als Pflege- und/oder Feuchthaltewirkstoffe werden, wie bereits oben erläutert, erfindungsgemäß Wirkstoffe verstanden, die nach dem Verdunsten der Alkoholkomponente auf der Haut verbleiben. Hierunter fallen auch übliche Hautpflegestoffe wie z.B. Dexpanthenol, Glyzerin, 1,2-Propandiol, Sorbitol, 1,3- Butylenglykol, Polyethylenglykol und andere Polyalkohole, Hyaluronsäuren, Harnstoff, Kamillenextrakte, oder alkokylierte Cetylalkohole (z.B. Procetyl AWS - Produkt der Fa. CRODA GmbH, Nettetal-Kaldenkirchen, Deutschland) etc. und/oder nichtflüchtige antimikrobiell wirksame Substanzen und gegebenenfalls weitere Hilfsstoffe wie z.B. hydrierte ethoxylierte Rizinusöle z.B. Cremophor® RH 40 (Fa. BASF AG).

Diese Pflege- und/oder Feuchthaltewirkstoffe können in den Endprodukten allein oder in Kombination üblicherweise in Mengen von 0 bis 5 Gew.-%, vorzugsweise 0,1 bis 3 Gew.-% und besonders bevorzugt 2 bis 3 Gew.-% bezogen auf das Gesamtgewicht der Haut- und Handpflegegele enthalten sein.

Da der hohe Alkoholanteil in den Haut- und Handpflegegelen beim Auftragen eine Austrocknung der damit behandelten Hautpartien bewirkt, ist der Einsatz von mindestens einem Hautpflegestoff und/oder ein Feuchthaltewirkstoff im Hinblick auf die häufige Applikation solcher Desinfektionsmittel in der täglichen Praxis eigentlich unabdingbar.

Vorzugsweise wird für das erfindungsgemäße Verfahren eine Pflegekomponente bestehend aus Glyzerin, 1,2-Propandiol, Dexpanthenol, hydriertem ethoxylierten Rizinusöl (Anzahl der EO-Einheiten 40) und alkoxylierter Cetylalkohol, und besonders bevorzugt eine Kombination aus Dexpanthenol, Glyzerin und/oder 1,2-Propandiol als Feuchthaltemittel und PPG-5-Ceteth-20 als Emollient eingesetzt.

Der Zusatz von nichtflüchtigen antimikrobiell wirksamen Substanzen dient dazu, die desinfizierenden Eigenschaften der Alkoholkomponente zu verstärken. Jedoch ist nach dem erfindungsgemäßen Verfahren der Zusatz solcher antimikrobieller Wirkstoffe nicht notwendig, da die nach dem Verfahren hergestellten stabilen Haut- bzw. Handpflegegele einen so hohen Alkoholgehalt aufweisen, so daß die Alkoholkomponente der resultierenden Gele allein als desinfizierender Wirkstoff dient.

Als einzusetzende antimikrobiell wirksame Substanzen, die vorzugsweise allein oder als Kombination mehrer desinfizierender Wirkstoffe in den erfindungsgemäßen Haut-und Handpflegegelen enthalten sind, sind insbesondere zu nennen Invertseifen, wie z.B. Kationtenside, Quartäre Ammoniumverbindungen u.a. Benzalkoniumchloride oder Benzethoniumchlorid, Biguanidverbindungen wie z.B. Chlorhexidin-Salze, Phenolverbindungen, Kresole, Perverbindungen, Iodverbindungen, z.B. Polyvidon-Iod, organische Säuren etc. Diese zusätzlich einzusetzenden Wirkstoffe können üblicherweise in Mengen von 0 bis 2 Gew.-% bezogen auf das Gesamtgewicht der Haut- und Handpflegegele enthalten sein, wobei der Fachmann die Einsatzmenge so wählt, daß es zu keiner Beeinträchtigung der Gelbildung kommt.

Die Haut- und Handpflegegele können gegebenenfalls Hilfs- bzw. Zusatzstoffe wie z.B. Farbstoffe, Lösungsvermittler, Tenside, Komplexbildner, Sequestrierungsmittel, Lichtschutzfilter oder Parfümstoffe enthalten, insbesondere in üblichen Mengen von vorzugsweise 0,05 bis 5 Gew.-% .-% bezogen auf das Gesamtgewicht der Haut- und Handpflegegele.

Vorteilhaft ist weiterhin die Zugabe von natürlichen pflanzlichen Gerbstoffen wie z.B. Frauenmantelkraut (Alchemilla xanthochlora, Rosaceae), Tormentillwurzelstock (potentilla erecta, Rosaceae), Eichenrinde (Quercus petraea und Quercus robur, Fagaceae) Ratanhiawurzel (Krameria lappacea syn. K. triandra, Krameriaceae), Hamamelisblätter (Hamamelis virginiana, Hamamelidaceae) und Blaubeeren (Vaccinium myrtillus, Ericaceae) und natürlichen synthetischen Gerbstoffen, wie z.B. Na- bichlorophenylsulfamin, vorzugsweise in einer Menge von 0,01 bis 5 Gew.-% Aktivsubstanz, bezogen auf die Gesamtmenge der Haut- und Handpflegegele, wobei Hamamelis virginiana als Gerbstoff besonders bevorzugt ist.

Schließlich enthalten die nach dem erfindungsgemäßen Verfahren hergestellten Haut-und Handpflegegele Wasser in einer Menge, so daß auch das resultierende Gel eine für den Anwendungszweck ausreichende Viskosität von 4000 bis 10 000 Centipoise (4 bis 10 Pa·s bei 25 °C) aufweist. Hierbei sollte ein Wassergehalt von 10 Gew.-% bezogen auf das Gesamtgewicht der Haut- und Handpflegegele nicht unterschritten werden, um die Gelbildung nicht zu behindern. Das Wasser wird vorzugsweise in deionisierter Form eingesetzt.

In einer bevorzugten Ausführungsform des Verfahrens wird die Menge des vollentsalzten, d.h. deionisierten Wassers vorgelegt und das Acrylsäurepolymerisat, vorzugsweise CARBOPOL® ETD 2020 und/oder CARBOPOL® ETD 2001 wird hierin dispergiert, so daß eine milchigtrübe Dispersion entsteht.

Zu dieser wässrigen Lösung wird nun eine alkoholische Pflegephase hinzugefügt. Hierbei handelt es sich um eine alkoholische Lösung der einzusetzenden Pflege- und/oder Feuchthaltewirkstoffe, vorzugsweise mindestens eines Hautpflegestoffs und/oder Feuchthaltewirkstoffes und gegebenenfalls antimikrobielle Substanzen, Lösungsvermittler sowie weitere übliche Hilfsstoffe. Vorzugsweise sind die einzusetzenden Hautpflege- und/oder Feuchthaltewirkstoffe in 15 bis 30 Gew.-% der Alkoholgesamtmenge des Geles gelöst.

Nach der Zugabe dieser alkoholischen Pflegephase und anschließendem Rühren, bis eine homogene Lösung entsteht, wird dann der resultierenden Lösung 30 bis 60 Gew.-%, vorzugsweise 45 bis 55 Gew.-% der Alkoholgesamtmenge des Geles zugegeben und homogen untergerührt.

In einem weiteren Schritt wird nun die erhaltene Lösung bzw. das in der Lösung enthaltene Acrylsäurepolymerisat mit wenigstens einem Neutralisationsmittel neutralisiert. Als Neutralisationsmittel werden übliche basische anorganische und / oder organische Verbindungen, insbesondere organische Amine verwendet. Vorzugsweise werden Isopropylamin, Aminomethylpropanol, Aminomethylpropandiol, Triethanolamin sowie Diisopropanolamin, besonders bevorzugt Tetrahydroxypropylethylendiamin eingesetzt bis ein pH-Wert von 6 bis 9, vorzugsweise 7 bis 8 im Produkt erreicht ist. Bevorzugt wird das Neutralisationsmittel in 15 bis 30 Gew.-% der Alkoholgesamtmenge des Geles gelöst und der Acrylsäurepolymerisatösung zur Gelbildung hinzugefügt, da so eine niedrigviskose, gut zu verarbeitende Lösung erhalten werden kann. Diese Lösung darf jedoch nur sehr kurz homogen untergerührt werden, da die resultierende Lösung jetzt scherempfindlich ist.

Abschließend wird mindestens 5 Gew.-% bezogen auf die Gesamtmenge des Geles wenigstens eines Alkohols der allgemeinen Formel

**R-OH**

in der R für einen aliphatischen, linearen oder verzweigten Kohlenwasserstoffrest mit 3 bis 8 Kohlenstoffatomen steht, zur Gesamtmischung gegeben werden und homogen untergerührt. Die Zugabe dieses Alkohols, hierbei ist Isopropylalkohol besonders bevorzugt, hat kontrolliert zu erfolgen. Eine zu schnelle Zugabe bewirkt irreversible Trübungen bzw. die gewünschte Gelstabilität wird nicht erreicht, weil die Viskosität des hergestellten Gels zu stark abfällt.

Es sei angemerkt, daß die bevorzugt eingesetzten Acrylsäurepolymerisate vom CARBOPOL®-Typ, insbesondere CARBOPOL® ETD 2020 und/oder CARBOPOL® ETD 2001 zunächst in dem deionisierten Wasser dispergiert werden müssen. Ein Lösen, Dispergieren bzw. das Vorlegen des Alkohols der allgemeinen Formel R-OH, insbesondere des Isopropylalkohols ist nicht möglich, da eine anschließende Neutralisation nicht zur Gelbildung führt.

Es ist auch möglich, die alkoholische Wirkstoffkomponente nicht, wie oben dargestellt, fraktioniert zur wäßrigen Acrylsäurepolymerisatlösung zu geben, sondern die Hauptkomponente der alkoholischen Wirkstoffkomponente, vorzugsweise technischer Alkohol vorab zusammen mit den Hautpflege- und/oder Feuchthaltewirkstoffen komplett zur wässrigen Acrylsäurepolymerisatdispersion zu geben und anschließend insbesondere mit Tetrahydroxypropylethylendiamin, Isopropylamin oder Aminomethylpropanol, die in Isopropylalkohol gelöst sein können, zu neutralisieren.

Es ist jedoch zwingend notwendig, wie zuvor beschrieben, den Alkohol der o.g. Formel R-OH erst nach oder während, d.h. der hinzuzufügende Alkohol der o.g. Formel R-OH wird zuvor mit dem Neutralisationmittel vermischt, der Neutralisation kontrolliert zuzusetzen, da es sonst zu keiner stabilen Gelbildung kommt, weil das gebildete Gelgerüst, insbesondere durch zu heftiges Rühren wieder zerstört wird.

Um die Gelbildung nicht zu beeinträchtigen, sollte mindestens ein Wassergehalt von 10 Gew.-% bezogen auf das Gesamtgewicht des Geles vorliegen und / oder ein Alkoholanteil des Alkohols der o.g. Formel R-OH von höchstens 20 Gew.-% bezogen auf das Gesamtgewicht des Geles % nicht überschritten werden.

Da das erfindungsgemäße Verfahren vornehmlich zur Herstellung von desinfizierenden Haut- und Handpflegegelen bestimmt ist, die die oben genannten Normen erfüllen sollen, z.B. darf die desinfizierende Wirksamkeit nach 30 Sekunden Einwirkzeit nicht beeinträchtigt sein, muß der gesamte Alkoholanteil der alkoholischen Wirkstoffkomponente bezogen auf das Gesamtgewicht der Haut- und Handpflegegele, der vorzugsweise eine Mischung aus Ethanol (technisch) und 2-Propanol ist, bei mindestens 60 Gew.-% liegen, wobei der Anteil des Alkohols der o.g. Formel R-OH 5 Gew.-% nicht unterschreiten darf.

Die Vorteile des erfindungsgemäßen Verfahrens sind, daß hierdurch stabile desinfizierende Haut- und Handpflegegelen mit hohem Alkoholgehalt, insbesondere desinfizierenden Handpflegegelen mit pflegenden Komponenten erhältlich sind, die ohne weitere zusätzliche antimikrobielle Zusatzstoffe u.a. die Normen

| | |
|---|---|
| DIN EN 1499: | Desinfizierende Händewaschung |
| DIN EN 1500 | Hygienische Händedesinfektion |

erfüllen und darüber hinaus eine Hepatitis B Wirkung aufweisen. Insbesondere die letztere Wirkung ist besonders vorteilhaft, da das Hepatitis B-Virus wie das HIV-Virus, das für die Verbreitung von AIDS (Acquired Immune Deficiency Syndrome) verantwortlich ist, übertragen wird, aber stabiler und infektiöser als das HIV-Virus ist. Daher sind alle Vorsichtsmaßnahmen gegen eine Übertragung von Hepatitis B auch präventiv gegen das HIV-Virus (vgl. Deutsches Ärzteblatt 84, Heft 18, S. B 874 vom 30.04.1987).

Darüber hinaus werden in dem erfindungsgemäßen Verfahren die einzelnen Komponenten und insbesondere die Pflege- und/oder Feuchthaltewirkstoffe bei Raumtemperatur zur Reaktion gebracht, so daß durch dieses Herstellungsverfahren im Vergleich zu üblichen im Stand der Technik bekannten Verfahren Energiekosten eingespart werden, was das erfindungsgemäße Verfahren natürlich sehr wirtschaftlich macht.

Bevorzugte Haut- und Handpflegegele haben folgende Zusammensetzungen:

| | |
|---|---|
| Alkoholkomponente | 60 bis 90 Gew.-% |
| Pflege- und/oder Feuchthaltewirkstoff | 0 bis 5 Gew.-% |
| Wasser | 10 bis 35 Gew.% |
| Verdickungsmittel | 0,2 bis 3 Gew.-% |
| Hilfs-/Zusatzstoffe | 0 bis 5 Gew.-% |

## Patentansprüche

1. Verfahren zur Herstellung von desinfizierenden Haut- und Handpflegegelen, die
a.) mindestens 60 Gew.-%, bezogen auf die Gesamtmenge des Geles, einer alkoholischen Wirkstoffkomponente bestehend aus einer Alkoholmischung, die einen Anteil von mindestens 5 Gew.-%, bezogen auf die Gesamtmenge des Geles, wenigstens eines Alkohols der allgemeinen Formel
**R-OH**
in der R für einen aliphatischen, linearen oder verzweigten Kohlenwasserstoffrest mit 3 bis 8 Kohlenstoffatomen steht, enthält,
b.) mindestens ein Verdickungsmittel auf Basis von Polyacryl- und Polymethacrylsäureverbindungen sowie gegebenenfalls weiteren Verdickungsmitteln
c.) 0 bis 5 Gew.-%, bezogen auf die Gesamtmenge des Geles, mindestens einen Pflege-und/oder Feuchthaltewirkstoff sowie
d.) Wasser enthalten,
wobei
1.) mindestens ein Acrylsäurepolymerisat zu einer Vorlage von Wasser gegeben und anschließend das Polymerisat dispergiert wird, bis eine milchigtrübe, homogene Dispersion entsteht;
2.) eine alkoholische Pflegephase, enthaltend eine alkoholische Lösung sowie gegebenenfalls Pflege- und/oder Feuchthaltewirkstoffe, fraktioniert oder komplett zur Dispersion hinzugefügt wird,
3.) die gebildete Polymerisatlösung mit mindestens einem Neutralisationsmittel neutralisiert wird,
**dadurch gekennzeichnet, daß** mindestens 5 Gew.-%, bezogen auf die Gesamtmenge des Geles, wenigstens eines Alkohols der allgemeinen Formel
**R-OH**
in der R für einen aliphatischen, linearen oder verzweigten Kohlenwasserstoffrest mit 3 bis 8 Kohlenstoffatomen steht, nach oder während der Neutralisation zur Gesamtmischung gegeben werden, und die Menge des eingesetzten Verdickungsmittel so gewählt wird, daß das resultierende Haut- und Handpflegegel eine Viskosität von 4 bis 10 Pa·s bei 25° C (4000 bis 10 000 Centipoise) aufweist.

2. Verfahren nach Anspruch 1, **dadurch gekennzeichnet, daß** die alkoholische Wirkstoffkomponente eine Hauptkomponente aus Methanol, Ethanol, n- Propanol, Isopropylalkohol oder eine Mischung hiervon aufweist, und die mindestens 5 Gew.-%, bezogen auf die Gesamtmenge des Geles, wenigstens eines Alkohols der allgemeinen Formel
**R-OH**
in der R für einen aliphatischen, linearen oder verzweigten Kohlenwasserstoffrest mit 3 bis 8 Kohlenstoffatomen steht, enthält.

3. Verfahren nach den Ansprüchen 1 bis 2, **dadurch gekennzeichnet, daß** die alkoholische Wirkstoffkomponente höchstens 20 Gew-%, bezogen auf die Gesamtmenge des Geles, eines Alkohols der allgemeinen Formel
**R-OH**
in der R für einen aliphatischen, linearen oder verzweigten Kohlenwasserstoffrest mit 3 bis 8 Kohlenstoffatomen steht, enthält.

4. Verfahren nach den Ansprüchen 1 bis 3, **dadurch gekennzeichnet, daß** als Alkohole der allgemeinen Formel
**R-OH**
in der R für einen aliphatischen, linearen oder verzweigten Kohlenwasserstoffrest mit 3 bis 8 Kohlenstoffatomen steht, 1-Propanol, 2-Propanol, 1-Butanol, Isobutylalkohol, tert. Butylalkohol, die Amylalkohole 1-, 2-, 3- Pentanol und/oder Neopentylalkohol, allein oder in Mischungen verwendet werden.

5. Verfahren nach den Ansprüchen 1 bis 4, **dadurch gekennzeichnet, daß** die alkoholische Wirkstoffkomponente ein Gemisch aus Ethanol und 2-Propanol ist.

6. Verfahren nach den Ansprüchen 1 bis 5, **dadurch gekennzeichnet, daß** mindestens ein Pflege- und/oder Feuchthaltewirkstoff in der alkoholischen Pflegephase in 15 bis 30 % der Alkoholgesamtmenge des Geles gelöst ist.

7. Verfahren nach den Ansprüchen 1 bis 6, **dadurch gekennzeichnet, daß** nach der Zugabe der alkoholischen Pflegephase der resultierenden Lösung 30 bis 60 Gew.-% der Alkoholgesamnnenge des Geles zugegeben wird.

8. Verfahren nach den Ansprüchen 1 bis 7, **dadurch gekennzeichnet, daß** die Polymerisatlösung mit mindestens einem Neutralisationsmittel auf einen pH-Wert von von 6 bis 9 neutralisiert wird.

9. Verfahren nach den Ansprüchen 1 bis 8, **dadurch gekennzeichnet, daß** das Neutralisationsmittel in 15 bis 30 Gew.-% der Alkoholgesamtmenge des Geles gelöst ist.

10. Verfahren nach den Ansprüchen 1 bis 9, **dadurch gekennzeichnet, daß** das resultierende Haut- und Handpflegegel 10 bis 30 Gew.-% Wasser enthält.

11. Verfahren nach den Ansprüchen 1 bis 10, **dadurch gekennzeichnet, daß** das resultierende Haut- und Handpflegegel 0,1 bis 3 Gew.-% mindestens eines Pflege- und/oder Feuchthaltewirkstoffs enthält.

12. Verfahren nach den Ansprüchen 1 bis 11, **dadurch gekennzeichnet daß** natürliche pflanzliche Gerbstoffe und/oder synthetische Gerbstoffe in einer Menge von 0,01 bis 5 Gew.-% Aktivsubstanz, im resultierenden Haut- und Handpflegegel enthalten sind.

## Claims

1. Method for producing disinfectant skin care and hand care gels which contain
a.) at least 60% by weight, based on the total amount of the gel, of an active alcoholic component consisting of an alcohol mixture which contains a fraction of at least 5% by weight, based on the total amount of the gel, of at least one alcohol of the general formula
**R-OH**
where R is an aliphatic, linear or branched hydrocarbon moiety having 3 to 8 carbon atoms,
b.) at least one thickener based on polyacrylic and polymethacrylic acid compounds and also optionally other thickeners
c.) 0 to 5% by weight, based on the total amount of the gel, of at least one care ingredient and/or humectant and also
d.) water,
wherein
1.) at least one acrylic acid polymer is added to a reservoir of water and subsequently the polymer is dispersed until a milky-opaque homogeneous dispersion forms;
2.) an alcoholic care phase containing an alcoholic solution and also optionally care ingredients and/or humectants is added in fractionated form or complete to the dispersion,
3.) the polymer solution formed is neutralized with at least one neutralizing agent,
**characterized in that** at least 5% by weight, based on the total amount of the gel, of at least one alcohol of the general formula
**R-OH**
where R is an aliphatic, linear or branched hydrocarbon moiety having 3 to 8 carbon atoms are added to the total mixture after or during the neutralization, and the amount of the thickener used is selected such that the resultant skin care and hand care gel has a viscosity of 4 to 10 Pa·s at 25°C (4000 to 10 000 centipoise).

2. Method according to Claim 1, **characterized in that** the active alcoholic component contains a main component of methanol, ethanol, n-propanol, isopropyl alcohol or a mixture thereof, and which contains at least 5% by weight, based on the total amount of the gel, of at least one alcohol of the general formula
**R-OH**
where R is an aliphatic, linear or branched hydrocarbon moiety having 3 to 8 carbon atoms.

3. Method according to Claims 1 to 2, **characterized in that** the active alcoholic component contains at most 20% by weight, based on the total amount of the gel, of an alcohol of the general formula
**R-OH**
where R is an aliphatic, linear or branched hydrocarbon moiety having 3 to 8 carbon atoms.

4. Method according to Claims 1 to 3, **characterized in that**, as alcohols of the general formula
**R-OH**
where R is an aliphatic, linear or branched hydrocarbon moiety having 3 to 8 carbon atoms, 1-propanol, 2-propanol, 1-butanol, isobutyl alcohol, tert-butyl alcohol, the amyl alcohols 1-, 2-, 3-pentanol and/or neopentyl alcohol are used alone or in mixtures.

5. Method according to Claims 1 to 4, **characterized in that** the active alcoholic component is a mixture of ethanol and 2-propanol.

6. Method according to Claims 1 to 5, **characterized in that** at least one care ingredient and/or humectant is dissolved in the alcoholic care phase in 15 to 30% of the total amount of alcohol of the gel.

7. Method according to Claims 1 to 6, **characterized in that**, after the addition of the alcoholic care phase, 30 to 60% by weight of the total amount of alcohol of the gel are added to the resultant solution.

8. Method according to Claims 1 to 7, **characterized in that** the polymer solution is neutralized with at least one neutralizing agent to a pH of 6 to 9.

9. Method according to Claims 1 to 8, **characterized in that** the neutralizing agent is dissolved in 15 to 30% by weight of the total amount of alcohol of the gel.

10. Method according to Claims 1 to 9, **characterized in that** the resultant skin care and hand care gel contains 10 to 30% by weight of water.

11. Method according to Claims 1 to 10, **characterized in that** the resultant skin care and hand care gel contains 0.1 to 3% by weight of at least one care ingredient and/or humectant.

12. Method according to Claims 1 to 11, **characterized in that** natural plant tannins and/or synthetic tannins are present in the resultant skin care and hand care gel in an amount of 0.01 to 5% by weight of active substance.

## Revendications

1. Procédé pour la préparation de gels désinfectants pour le soin de la peau et des mains, qui contiennent
a) au moins 60 % en poids, par rapport à la quantité totale du gel, d'un composant principe actif alcoolique constitué d'un mélange d'alcools qui contient une proportion d'au moins 5 % en poids, par rapport à la quantité totale du gel, d'au moins un alcool de formule générale
**R-OH**
dans laquelle R représente un radical hydrocarboné aliphatique, linéaire ou ramifié ayant de 3 à 8 atomes de carbone,
b) au moins un épaississant à base de composés de type poly(acide acrylique) et poly(acide méthacrylique) ainsi qu'éventuellement d'autres épaississants,
c) 0 à 5 % en poids, par rapport à la quantité totale du gel, d'au moins une substance active hydratante et/ou de soin, ainsi que
d) de l'eau,
dans lequel
1) on ajoute un polymère d'acide acrylique à de l'eau disposée au préalable et on disperse ensuite le polymère, jusqu'à l'obtention d'une dispersion homogène trouble comme le lait ;
2) on ajoute par fractions ou en totalité à la dispersion une phase alcoolique de soin, contenant une solution alcoolique ainsi qu'éventuellement des substances actives hydratantes et/ou de soin,
3) on neutralise avec au moins un agent de neutralisation la solution de polymère formée,
**caractérisé en ce qu'**on ajoute au mélange total, avant ou après la neutralisation, au moins 5 % en poids, par rapport à la quantité totale du gel, d'au moins un alcool de formule générale
**R-OH**
dans laquelle R représente un radical hydrocarboné aliphatique, linéaire ou ramifié ayant de 3 à 8 atomes de carbone, et la quantité de l'épaississant utilisé est choisie de manière que le gel pour le soin de la peau et des mains qui en résulte présente une viscosité de 4 à 10 Pa.s à 25°C (4 000 à 10 000 centpoises).

2. Procédé selon la revendication 1, **caractérisé en ce que** le composant principe actif alcoolique comporte un composant principal à base de méthanol, éthanol, n-propanol, alcool isopropylique ou d'un mélange de ceux-ci, et qui contient au moins 5 % en poids, par rapport à la quantité totale du gel, d'au moins un alcool de formule générale
**R-OH**
dans laquelle R représente un radical hydrocarboné aliphatique, linéaire ou ramifié ayant de 3 à 8 atomes de carbone.

3. Procédé selon les revendications 1 et 2, **caractérisé en ce que** le composant principe actif alcoolique contient au maximum 20 % en poids, par rapport à la quantité totale du gel, d'un alcool de formule générale
**R-OH**
dans laquelle R représente un radical hydrocarboné aliphatique, linéaire ou ramifié ayant de 3 à 8 atomes de carbone.

4. Procédé selon les revendications 1 à 3, **caractérisé en ce qu'**en tant qu'alcools de formule générale
**R-OH**
dans laquelle R représente un radical hydrocarboné aliphatique, linéaire ou ramifié ayant de 3 à 8 atomes de carbone, on utilise le 1-propanol, le 2-propanol, le 1-butanol, l'alcool isobutylique, l'alcool tert-butylique, les alcools amyliques 1-, 2-, 3-pentanol et/ou alcool néopentylique, seuls ou en mélanges.

5. Procédé selon les revendications 1 à 4, **caractérisé en ce que** le composant principe actif alcoolique est un mélange d'éthanol et de 2-propanol.

6. Procédé selon les revendications 1 à 5, **caractérisé en ce que** dans la phase alcoolique de soin au moins une substance active hydratante et/ou de soin est dissoute dans 15 à 30 % de la quantité totale d'alcool du gel.

7. Procédé selon les revendications 1 à 6, **caractérisé en ce qu'**après l'addition de la phase alcoolique de soin on ajoute à la solution résultante de 30 à 60 % en poids de la quantité totale d'alcool du gel.

8. Procédé selon les revendications 1 à 7, **caractérisé en ce que** la solution de polymère est neutralisée à un pH de 6 à 9 avec au moins un agent de neutralisation.

9. Procédé selon les revendications 1 à 8, **caractérisé en ce que** l'agent de neutralisation est dissous dans 15 à 30 % en poids de la quantité totale d'alcool du gel.

10. Procédé selon les revendications 1 à 9, **caractérisé en ce que** le gel résultant pour le soin de la peau et des mains contient de 10 à 30 % en poids d'eau.

11. Procédé selon les revendications 1 à 10, **caractérisé en ce que** le gel résultant pour le soin de la peau et des mains contient de 0,1 à 3 % en poids d'au moins une substance active hydratante et/ou de soin.

12. Procédé selon les revendications 1 à 11, **caractérisé en ce que** des tanins végétaux naturels et/ou des tanins synthétiques sont contenus, en une quantité de 0,01 à 5 % en poids de substance active, dans le gel résultant pour le soin de la peau et des mains.
